# EUROPEAN PATENT SPECIFICATION

(11) **EP 3 558 227 B1**
(45) Date of publication and mention of the grant of the patent: **10.11.2021**
(21) Application number: 16826064.4
(22) Date of filing: 23.12.2016
(51) Int. Cl.: A61K 8/34, A61Q 17/04, A61K 8/81, A61K 8/898, A61K 8/11, A61K 8/73, A61Q 17/00

(54) **SILICONE-IN-WATER COSMETIC**
SILIKON-IN-WASSER-KOSMETIKUM
COMPOSITION COSMÉTIQUE SILICONE-DANS-EAU

(43) Date of publication of application: 30.10.2019
(73) Proprietor: LVMH Recherche, 45800 Saint Jean de Braye (FR)
(72) Inventor: KIKUCHI, Masahiro, Tokyo 102-0092 (JP); OZAWA, Mai, Tokyo 102-0092 (JP); SAKODA, Takayoshi, Tokyo 102-0092 (JP)
(74) Representative: Cabinet Beau de Loménie
(86) International application number: PCT/EP2016/082643
(87) International publication number: WO 2018/114014

(56) References cited:
- US-A1- 2010 129 305
- US-A1- 2015 272 861

## Description

### Technical Field

The present invention relates to a silicone-in-water cosmetic.

### Background Art

Cosmetic products comprising a polyhydric alcohol such as glycerin for use in skin care are liked by many consumers, since they possess a nourishing and hydrating effect. Some of these products contain only oils and lack a refreshing effect on skin, have a heavy sensation, an oily sensation and a slippery sensation. Other products containing glycerin have been developed in the form of watery products that provide the skin with a great deal of fresh watery sensation, have a low nourishing effect and provides the skin with a sticky sensation. As for a cosmetic with combined merits of these two types, there has been proposed an emulsified cosmetic having an oil ingredient in combination with water.

There is a need to propose cosmetics comprising water and a polyhydric alcohol such as glycerin that simultaneously combine several sensory properties that are nourishing effect, fresh watery sensation, no sticky sensation and no slippery sensation on the skin.

There is also a need to still increase the nourishing effect of cosmetics comprising a polyhydric alcohol, one way thereof being to increase the polyhydric alcohol content. However, the inventors have found that a silicone-in-water cosmetic that is transparent and contains high percentages of glycerin surprisingly lack nourishing effect, lack fresh watery sensation; give sticky sensation and slippery sensation on the skin.

Additionally, emulsified cosmetics are most frequently turbid white, whereas products oily cosmetics and hydrogels look transparent to the consumer. Therefore, there is also a need for cosmetics being transparent. An improved transparency of oil-in-water cosmetics has been proposed in WO 2013/111441, US 2015/0272861, US 2010/129305 and JP 2004-51574. These emulsions contain surfactants, fatty compounds such as oils, and/or oil gelling agents such as waxes, and consequently lack satisfactory care and satisfactory sensation properties such as described here above.

Indeed, in order to impart a transparent appearance to an oil-in-water emulsified cosmetic, it is necessary to raise the contents of an oil ingredient and a polyhydric alcohol both of which have close refractive indexes; however, if the content of the oil ingredient is high, it cannot be stably dispersed in the emulsion. Therefore, as in the emulsified cosmetics of the prior art, considerable amounts of a surfactant and a gelling agent are required. There is nevertheless a problem that these additives produce irritation and a sticky sensation to the skin. On the other hand, if the content of the polyhydric alcohol is high, it will inevitably give an unpleasant slippery sensation to the skin.

The cosmetic of the present invention has solved such problems and provides a silicone-in-water cosmetic that simultaneously combines several sensory properties that are nourishing effect, fresh watery sensation, no sticky sensation and slippery sensation on the skin. In addition, such a silicone-in-water cosmetic is advantageously transparent or translucent, and stable over time. It is the first time that such a combination of advantages is obtained for a cosmetic.

### Description of Invention

According to an embodiment of the present invention, there is provided a transparent stable silicone-in-water cosmetic comprising water, at least a polyhydric alcohol, a polyorganosiloxane dispersion comprising encapsulated polyorganosiloxane particles, and a hydrophilic polymer that is preferably dissolved in the mixture of water and polyhydric alcohol, said polyorganosiloxane particles comprising a polyorganosiloxane core covered with a polymer shell, wherein the hydrophilic polymer is pullulan.

The wording "silicone-in-water" that is used according to the invention means a dispersion of solid silicone particles in water.

According to one embodiment of the present invention, there is provided a cosmetic comprising from 20 to 70% by weight of water, from 15% to 60% by weight of at least one polyhydric alcohol, from 5 to 25% by weight of polyorganosiloxane particles that comprise a polyorganosiloxane core covered with a polymer shell, said polyorganosiloxane particles being dispersed in water and said polyhydric alcohol,
wherein said silicone-in-water cosmetic further comprises from 0.001 to 5% by weight of at least one hydrophilic polymer that is pullulan,
the percentages being by weight of the weight of the cosmetic.

While the silicone-in-water cosmetic of the present invention gives an improved nourishing effect and a fresh watery sensation to the skin, it has the controlled sticky sensation and slippery sensation on the skin. The cosmetic also benefit from a translucent or transparent aspect, and is stable over time.

In the silicone-in-water cosmetic of the present invention, the content of the hydrophilic polymer is desirably from 0.001 to 5.0% by mass based on the total mass. If the content is set in such a range, the sticky sensation and slippery sensation on the skin will be markedly controlled.

The viscosity of the silicone-in-water cosmetic of the present invention is desirably from 250 to 100,000 mPa.s at 25 °C. If the viscosity is set in such a range, the polyorganosiloxane can be dispersed in the aqueous medium more stably.

### Advantageous Effects of Invention

According to the present invention, there can be provided a stable and transparent silicone-in-water cosmetic that gives a nourishing effect and a fresh watery sensation to the skin and that has a controlled sticky sensation and slippery sensation on the skin. The

### Description of Embodiments

The preferred embodiments of the present invention will be described below; however, the present invention shall not be limited to the embodiments below in any way.

The silicone-in-water cosmetic according to the present embodiment comprises (a) an aqueous medium comprising water and a polyhydric alcohol.

As the water, there can be used steam-distilled water that is derived from plants, which includes lavender water, rose water, and orange flower water, in addition to distilled water, purified water, hot spring water, or deep water.

As the polyhydric alcohol, there are mentioned a glycol such as 1, 3-propanediol, propylene glycol, butylene glycol, pentylene glycol, dipropylene glycol, or polyethylene glycol; a glycerol such as glycerin, diglycerin, or polyglycerin; and a sugar alcohol such as sorbitol. Among these, glycerin is preferable. One kind of the polyhydric alcohol may be used alone, or two or more kinds may be used in combination.

The (a) aqueous medium may further comprise a mono-valent alcohol (e.g., phenoxyethanol) that is soluble in water or the polyhydric alcohol.

The content of the (a) aqueous medium is preferably from 50 to 95% by mass, and more preferably from 55 to 90% by mass based on the total mass of the cosmetic. As used in the present specification, the term "based on the total mass" means the total mass of the silicone-in-water cosmetic as being the basis.

In the cosmetic, the mass ratio of water relative to the polyhydric alcohol is preferably from 0.30 to 8.0, more preferably from 0.30 to 6.0, more preferably from 0.60 to 3.0, and further preferably from 1 to 1.5.

The silicone-in-water cosmetic according to the present embodiment comprises an aqueous medium essentially comprising water and a polyhydric alcohol. Polyorganosiloxane particles are dispersed in this aqueous medium that advantageously forms a continuous phase.

The polyorganosiloxane particles, in particular the core thereof, may have siloxane bonds (Si-O-Si) and an organic group, such as an alkyl group or an aryl group, bonded to the silicon atom. Examples of the polyorganosiloxane include polydimethylsiloxane (dimethicone) and polymethylphenylsiloxane. The polyorganosiloxane may have a functional group such as an unsaturated group, an amino group, or a fluoro group. The specific examples of such a polyorganosiloxane include vinyldimethicone, amodimethicone, and trifluoropropyldimethicone. In addition, the polyorganosiloxane may be a cross-linked product of different kinds of polyorganosiloxanes; the specific examples of such a polyorganosiloxane include the dimethicone/vinyldimethicone crosspolymer. One kind of the polyorganosiloxane may be used alone, or two or more kinds may be used in combination.

The polyorganosiloxane particles having a polyorganosiloxane core covered with a polymer shell have preferably an average dimension being from 5 to 300 microns.

The wording "polyorganosiloxane particles" can also designate "encapsulated polyorganosiloxane polymer particles having a polyorganosiloxane core and a polymer shell, wherein the polymer shell is not made of a polyorganosiloxane polymer. Such polyorganosiloxane particles can advantageously disperse in water and a polyhydric alcohol, in a stable manner.

The polyorganosiloxane particles may be dispersed in a dispersion medium that may comprise water and/or a polyhydric alcohol, and preferably, comprises water and at least one polyhydric alcohol. As the polyhydric alcohol, there is mentioned the same as a polyhydric alcohol as described above. Among these, glycerin or pentylene glycol is preferable. One kind of the polyhydric alcohol may be used alone, or two or more kinds may be used in combination.

The polyorganosiloxane core can be encapsulated with a polymer shell. This encapsulation with a polymer shell, can be realized when a cationic water-soluble polymer dissolved in a solvent (e.g., a carboxyvinyl polymer INCI name Carbomer) can form coacervation with an anionic polymer (e.g., amodimethicone), and form a shell surrounding the core. For example, it is preferred that the shell comprises a carboxyvinyl polymer (such as a carbomer) and an amodimethicone. Encapsulated polyorganosiloxane particles are for example described in US 2015/0272861, and can be mixed with the other ingredients, during a process for preparing the cosmetic, in the form of a dispersion that will be described further below.

Encapsulated dimethicone/vinyldimethicone crosspolymer are preferable as polyorganosiloxane particles.

The content of the polyorganosiloxane particles is preferably from 5 to 25% by mass, and more preferably, from 10 to 20% by mass based on the total mass of the cosmetic. The content of the polyorganosiloxane particles can be between two values that are selected in the group consisting of 5, 10, 15, 20 and 25% by weight of the weight of the cosmetic.

The content of the polyhydric alcohol is preferably from 15% to 60%, for example from 30 to 45% by mass based on the total mass of the cosmetic. The content of the polyhydric alcohol can be between two values that are selected in the group consisting of 15, 20, 25, 30, 35, 40, 45, 50, 55 and 60% by weight of the weight of the cosmetic. A content of polyhydric alcohol below 15% produces an opaque cosmetic with low nourishing properties. A content of polyhydric alcohol above 60% produces a slippery, opaque and oily cosmetic.

The content of water is preferably from 20 to 70% by weight, preferably from 35 to 55%, and more preferably from 40 to 50% by mass based on the total mass of the cosmetic. The content of water can be between two values that are selected in the group consisting of 20, 25, 30, 35, 40, 45, 50, 55, 60, 65 and 70% by weight of the weight of the cosmetic. A content of water below 20% produces an opaque cosmetic with low fresh watery sensation. A content of water above 70% produces a low nourishing and a non-sufficient fresh sensation.

The silicone-in-water cosmetic according to the present embodiment comprises a hydrophilic polymer, in addition to the aqueous medium and the polyorganosiloxane dispersion. As used in the present specification, the term "hydrophilic polymer" means a polymer that either dissolves in water or is swollen in water, when used in an amount as described above.

It is preferred that the pullulan is one having a weight average molecular weight of 5,000 or more.

Pullulan can be an extracellular polysaccharide, excreted by the polymorphic fungus *Aureobasidium pullulans.* The molecule is a linear a-D-glucan comprised of regular repeating trisaccharide units. The trisaccharide units are maltotrioses in which three glucose units are linked through 1,4-glucosidic bonds. Each maltotriose unit is terminally linked to a series of three other maltotrioses through a 1,6-glucosidic bonds creating a long stair-step-type structure. The molecular weight for the Pullulan may range from 50,000 to 500,000 daltons, depending upon the conditions under which the organism is grown.

One kind of the hydrophilic polymer may be used alone, or two or more kinds may be used in combination.

The hydrophilic polymer chiefly contributes to the effect of controlling the sticky sensation and slippery sensation on the skin in the silicone-in-water cosmetic, and its content is desirably from 0.001 to 5.0% by mass based on the total mass.

From the standpoint of more markedly controlling the sticky sensation of the silicone-in-water cosmetic on the skin, the content of the hydrophilic polymer in the silicone-in-water cosmetic is more preferably between two values that are selected from the group 0.001, 0.01, 0.02, 0.03, 0.05, 0.1, 0.3, 0.5, 1.0, 1.2, 1.4, 1.6, 1.8, 2.0, 2.2, 2.4, 2.6, 2.8, 3.0, 3.2, 3.4, 3.6, 3.8, 4.0, 4.5, and 5.0% by mass, for example from 0.03 to 2.0% by mass, and preferably from 0.05 to 2.0% by mass based on the total mass of the cosmetic.

The viscosity of the silicone-in-water cosmetic according to the present embodiment can be within the range of from 250 to 100,000 mPa.s at 25°C. By letting the viscosity range be within such a range, it is possible to disperse the polyorganosiloxane in the aqueous medium more stably. From the standpoint of dispersing the polyorganosiloxane in the aqueous medium far more stably, the viscosity of the silicone-in-water cosmetic at 25 °C is more preferably from 500 to 50,000 mPas, and more preferably, from 1,000 to 25,000 mPas, for example from 1,500 to 2500 mPa.s. The viscosity can be measured using a rotational rheometer (e.g., Rheolab QC; manufacture by Anton Paar GmbH) in the conditions that are known from one skilled in the art.

The silicone-in-water cosmetic may further include other compounds such as an oil agent, an UV absorber, an antioxidant, a brown-discoloration preventer, a preservative, a biological skin active agent, a stabilizer, a dye, a perfume, and the like, in addition to the above-described ingredients.

The cosmetic of the invention contains advantageously very low amounts of a surfactant, very low amounts of oils, very low amounts of a gelling agent for an oil, and/or very low amounts of solid particles other than polyorganosiloxane particles as described above. A very "low amount" means in the context of the invention, less than 5% by weight, preferably less than 3% by weight, most preferably less than 1%, or even less than 0,1% or 0.05% by weight of the weight of the cosmetic. According to a preferred embodiment, the cosmetic contains no surfactant, no oil, no gelling agent for an oil and/or no solid particles other than polyorganosiloxane particles as described above. The silicone-in-water cosmetic according to the present invention preferably essentially contains water, polyhydric alcohol, polyorganosiloxane particles and hydrophilic polymer; by "essentially", it is meant that mixture of water, polyhydric alcohol, polyorganosiloxane particles and hydrophilic polymer represent an amount being at least 90%, preferably at least 95% by weight, still preferably at least 99% by weight of the weight of the cosmetic.

The present invention is also concerned with a process for preparing a transparent silicone-in-water cosmetic as disclosed here above, said process comprising a step of preparing (a) an aqueous medium by mixing water and a first polyhydric alcohol, a step of adding (b) a polyorganosiloxane dispersion of polyorganosiloxane particles dispersed in water and a second polyhydric alcohol that can be identical or different from the first polyhydric alcohol, to the aqueous medium, wherein a difference in refractive index between the (a) aqueous medium and the (b) polyorganosiloxane dispersion is within ±0.05, or preferably within ±0.01.

In the (a) aqueous medium, the amount of water relative to the polyhydric alcohol is preferably from 0.20 to 10.0 parts by mass, more preferably from 0.30 to 5.0 parts by mass, and further preferably, from 0.40 to 4.0 parts by mass.

The content of the polyorganosiloxane dispersion that is used to prepare the cosmetic and that is mixed with the other components in order to prepared said cosmetic is preferably from 20 to 60% by mass, and more preferably from 25 to 55% by mass, or from 30 to 50% by mass based on the total mass of all the ingredients used to prepare the cosmetic. Further, in the silicone-in-water cosmetic, the mass ratio between the (b) polyorganosiloxane dispersion relative to the (a) aqueous medium that is mixed with said dispersion is preferably from 0.05 to 1.0 part by mass, and more preferably, from 0.10 to 0.80 parts by mass. The specific examples of a polyorganosiloxane dispersion include a dispersion of an encapsulated dimethicone/vinyldimethicone crosspolymer (product name: CES-1104®; manufactured by NuSil Technology LLC) and a dispersion of trifluoropropyldimethicone (product name: CES-3401®; manufactured by NuSil Technology LLC).

Additionally, the mass ratio of the hydrophilic polymer relative to the (a) aqueous medium is preferably from 0.00001 to 0.05 parts by mass, more preferably from 0.0001 to 0.05 parts by mass, and further preferably, from 0.0005 to 0.05 parts by mass.

The production of the silicone-in-water cosmetic according to the present embodiment can be carried out in the following manner. Specifically, water and the polyhydric alcohol are stirred and mixed so to be homogeneous and thereby, to obtain an aqueous medium; and to this are added a polyorganosiloxane dispersion raw material (comprising polyorganosiloxane particles covered with a polymer shell in a dispersion medium) and the hydrophilic polymer at the same time or at separate timings, and the whole is stirred and mixed until it becomes homogeneous. When the silicone-in-water cosmetic comprises the above-described additives, their additions are possible at any stage of the production.

In the silicone-in-water cosmetic according to the present embodiment, the difference in refractive index between the (a) aqueous medium and the (b) polyorganosiloxane dispersion is preferably within ±0.05, preferably ±0.01. By letting the difference in refractive index be within such a range, it is possible to render the silicone-in-water cosmetic translucent or transparent. It is possible to adjust the difference in refractive index within ±0.05 by appropriately setting the compounding ratio of the (b) polyorganosiloxane dispersion to the (a) aqueous medium, the mass ratio between water and the polyhydric alcohol in the aqueous medium, as described previously. The refractive index can be measured using a refractometer (e.g., Abbemat 550; manufactured by Anton Paar GmbH).

The polyorganosiloxane dispersion raw material is mixed with the other components (such as a polyhydric alcohol, water and a hydrophilic polymer) to form the cosmetic. Such a polyorganosiloxane dispersion is used as an ingredient to be mixed with the other ingredients used to prepare the cosmetic.

In particular, the polyorganosiloxane dispersion, which is mixed with water and polyhydric alcohol, is preferably in the form of a dispersion comprising a polyhydric alcohol, wherein said polyhydric alcohol is preferably identical to the polyhydric alcohol previously stirred and mixed with water. The polyorganosiloxane dispersion raw material also preferably contains another polyhydric alcohol such as pentylene glycol. The dispersion may also comprise additional compounds such as a dimethicone, a preservative, sodium hydroxide and disodium EDTA.

The polyorganosiloxane particles may preferably comprise a core encapsulated material that is covered with a polymer shell, said polymer shell being obtained from an anionic polymer and from a cationic polymer. The anionic polymer can be a polyacrylic polymer such as a carbomer, the cationic polymer can be an amine functionalized polyorganosiloxane such as an amodimethicone, and the core encapsulated material can be a polyorganosiloxane such as a dimethicone/vinyl dimethicone crosspolymer.

The silicone-in-water cosmetic according to the present embodiment is, for example, applicable as a lotion, a serum, a hydrogel or the like. One skilled in the art can easily determine that polyorganosiloxane particles are dispersed in a water phase or not. A water phase, in the context if the present invention, forms a continuous phase in the cosmetic.

### EXAMPLES

The silicone-in-water cosmetic will be further described herein below by way of the examples of the present invention; however, the present invention is not limited to the examples below.

### Test Examples: Silicone-in-Water Cosmetics

Water, glycerin, and phenoxyethanol were stirred and mixed so to be homogeneous and thereby, to obtain an aqueous medium that was heated to a temperature being from 50 to 60°C. The hydrophilic polymer according to the invention being Pullulan (manufactured by Hayashibara Co. Ltd.),a hydrophilic polymer according to the prior art being hydroxyethyl acrylate/sodium acryloyldimethyltaurate copolymer (product name: SIMULGEL® NS; manufactured by Seppic), and the polyorganosiloxane dispersion (product name: CES-1104®; manufactured by NuSil Technology LLC) were poured into the aqueous medium in this order. The obtained mixture was cooled to be 30 °C, stirred and mixed until it became homogeneous to prepare the respective cosmetics (Example 1, Example 2, Comparative Example 3, Comparative Example 4, and Comparative Example 1) as shown in Table below. The compounding ratios (% by mass) of the respective ingredients that were mixed are as shown in the Table below.

### (1) Transparency evaluation and Refractive index measurement

An appropriate amount of each cosmetic prepared above was dropped on a plate with described letters which were clearly, visually recognizable, and the evaluation was made according to the standard below.
Transparent: The letters can be visually recognized clearly.
Translucent: The letters can be visually recognized.
Opaque: The letters cannot be visually recognized.

The refractive indexes of the aqueous medium and the polyorganosiloxane dispersion were measured using a refractometer (Abbemat 550; manufactured by Anton Paar GmbH). The difference between the refractive index of the aqueous medium (consisting of water, glycerin and phenoxyethanol) and the refractive index of the polyorganosiloxane dispersion (CES-1104®) was within ±0.01 for examples 1, 2, 3 and 4.

### (2) Viscosity measurement

The viscosity of each cosmetic prepared above was measured using a rotational rheometer [e.g., Rheolab QC; manufactured by Anton Paar GmbH; a revolution rate of 50 rpm (3 minutes)].

### (3) Stability evaluation

Cosmetics of Examples 1, 2 and Comparative Example 1 were each accommodated in a two transparent containers that were sealed with a lid, and stored at 50 °C and 4 °C for one month. After storage, the absence or presence of the separation between an oil phase and a water phase was observed. That for which no separation between an oil phase and a water phase was observed both at 50 °C and at 4 °C was evaluated "Y"; and that for which the separation between an oil phase and a water phase was observed either at 50 °C or at 4 °C was evaluated "N."

### (4) Sensory evaluation

The nourishing effect, fresh watery sensation, lack of sticky sensation and lack of slippery sensation of each cosmetic prepared above were evaluated in a single use test by a cosmetic-specialized evaluation panel of the organization to which the present inventors belong, according to the standard below.

A: Noted; B: Average; C: Not noted

| Table | Proportions of ingredients that are mixed | Example 1 | Example 2 | Comparative Example 3 | Comparative Example 4 | Comparative Example 1 |
|---|---|---|---|---|---|---|
| (a) Aqueous medium | Water | 31,4 | 30,4 | 31,4 | 30,4 | 32,4 |
| | Glycerin | 27,2 | 27,2 | 27,2 | 27,2 | 27,2 |
| | Phenoxyethanol | 0,4 | 0,4 | 0,4 | 0,4 | 0,4 |
| (b) Polyorganosiloxane dispersion | Dimethicone (and) Water (and) Glycerin (and) Pentylene Glycol (and) Dimethicone/Vinyl Dimethicone Crosspolymer (and) Amodimethicone (and) Carbomer (and) Phenoxyethanol (and) Sodium Hydroxide (and) Disodium EDTA (CES-1104®) | 40,0 | 40,0 | 40,0 | 40,0 | 40,0 |
| Hydrophilic polymer | Pullulan | 1,0 | 2,0 | - | - | - |
| | Hydroxyethyl Acrylate/Sodium Acryloyldimethyl Taurate Copolymer (and) Squalane (and) Polysorbate 60 (SIMULGEL®NS) | - | - | 1,0 | 2,0 | - |
| Total (%) | | 100,0 | 100,0 | 100,0 | 100,0 | 100,0 |
| Viscosity (mPas) | | 1890 | 2341 | 7875 | 15510 | 1843 |
| Stability evaluation | | Y | Y | - | - | Y |
| Nourishing effect | | B | A | A | A | C |
| Fresh watery sensation | | A | A | A | B | C |
| Lack of sticky sensation | | A | A | A | A | C |
| Lack of slippery sensation | | A | A | A | A | C |

With respect to the cosmetic of Comparative Example 1 containing no hydrophilic polymer, the nourishing effect and fresh watery sensation were lacking, whereas the sticky sensation and slippery sensation were definitely noted. In contrast, as for the cosmetics of Examples 1 and 2 containing pullulan as the hydrophilic polymer, transparency, a good nourishing effect and fresh watery sensation were present, and at the same time, the sticky sensation and slippery sensation were controlled.

## Claims

1. A transparent or translucent silicone-in-water cosmetic comprising water, at least a polyhydric alcohol, a polyorganosiloxane dispersion comprising encapsulated polyorganosiloxane particles, and a hydrophilic polymer, said polyorganosiloxane particles comprising a polyorganosiloxane core covered with a polymer shell,
wherein the hydrophilic polymer is pullulan.

2. The silicone-in-water cosmetic according to Claim 1, wherein the cosmetic comprises an aqueous medium comprising water and the polyhydric alcohol, and the polyorganosiloxane particles are dispersed in this aqueous medium that forms a continuous phase.

3. The silicone-in-water cosmetic according to Claim 2, wherein the content of the aqueous medium is from 50% to 95% by mass based on the total mass of the cosmetic.

4. The silicone-in-water cosmetic according to Claim 1, wherein the polyhydric alcohol is a glycerol.

5. The silicone-in-water cosmetic according to Claim 1, wherein the mass ratio of water relative to the polyhydric alcohol is between 0.30 and 8.0.

6. The silicone-in-water cosmetic according to Claim 2, wherein the mass ratio between the polyorganosiloxane dispersion relative to the aqueous medium is 0.05 to 1.0 part by mass.

7. The silicone-in-water cosmetic according to Claim 1, wherein the polyorganosiloxane core is a dimethicone/vinyldimethicone crosspolymer, and the polymer shell comprises a cationic polymer and an anionic polymer.

8. The silicone-in-water cosmetic according to Claim 1, wherein the polyorganosiloxane particles amount is from 10% to 20% by weight.

9. The silicone-in-water cosmetic according to Claim 1, wherein polyhydric alcohol amount is from 15% to 60% by weight.

10. The silicone-in-water cosmetic according to Claim 9, wherein water amount is from 20% to 70% by weight.

11. The silicone-in-water cosmetic according to claim 1, having a viscosity of from 250 to 100,000 mPa.s at 25 °C.

12. The silicone-in-water cosmetic according to Claim 1, wherein the hydrophilic polymer content is from 0.001% to 5.0% by mass.

13. The silicone-in-water cosmetic according to Claim 1, wherein the mixture of water, polyhydric alcohol, polyorganosiloxane particles and hydrophilic polymer represent an amount being at least 90% by weight of the weight of the cosmetic.

14. A process for preparing a transparent or translucent silicone-in-water cosmetic according to claim 1, said process comprising a step of preparing (a) an aqueous medium by mixing water and a first polyhydric alcohol, a step of adding (b) a polyorganosiloxane dispersion of polyorganosiloxane particles dispersed in water and a second polyhydric alcohol that can be identical or different from the first polyhydric alcohol, to the aqueous medium, wherein a difference in refractive index between the (a) aqueous medium and the (b) polyorganosiloxane dispersion is within ±0.05, or preferably within ±0.01.

## Patentansprüche

1. Transparentes oder durchscheinendes Silikon-in-Wasser-Kosmetikum, das Wasser, mindestens einen polyhydrischen Alkohol, eine eingekapselte Polyorganosiloxan-Teilchen umfassende Polyorganosiloxan-Dispersion und ein hydrophiles Polymer umfasst, wobei die Polyorganosiloxan-Teilchen einen mit einer Polymerhülle bedeckten Polyorganosiloxan-Kern umfassen,
wobei das hydrophile Polymer Pullulan ist.

2. Silikon-in-Wasser-Kosmetikum nach Anspruch 1, wobei das Kosmetikum ein wässriges Medium umfasst, das Wasser und den polyhydrischen Alkohol umfasst, und die Polyorganosiloxan-Teilchen in diesem wässrigen Medium dispergiert sind, das eine kontinuierliche Phase bildet.

3. Silikon-in-Wasser-Kosmetikum nach Anspruch 2, wobei der Gehalt des wässrigen Mediums 50 bis 95 Masse-% bezogen auf die Gesamtmasse des Kosmetikums beträgt.

4. Silikon-in-Wasser-Kosmetikum nach Anspruch 1, wobei der polyhydrische Alkohol ein Glycerin ist.

5. Silikon-in-Wasser-Kosmetikum nach Anspruch 1, wobei das Massenverhältnis von Wasser in Bezug auf den polyhydrischen Alkohol zwischen 0,30 und 8,0 liegt.

6. Silikon-in-Wasser-Kosmetikum nach Anspruch 2, wobei das Massenverhältnis zwischen der Polyorganosiloxan-Dispersion in Bezug auf das wässrige Medium 0,05 bis 1,0 Masseteile beträgt.

7. Silikon-in-Wasser-Kosmetikum nach Anspruch 1, wobei der Polyorganosiloxan-Kern ein Dimethicon/Vinyldimethicon-Kreuzpolymer ist, und die Polymerhülle ein kationisches Polymer und ein anionisches Polymer umfasst.

8. Silikon-in-Wasser-Kosmetikum nach Anspruch 1, wobei die Menge der Polyorganosiloxan-Teilchen 10 bis 20 Gew.-% beträgt.

9. Silikon-in-Wasser-Kosmetikum nach Anspruch 1, wobei die Menge des polyhydrischen Alkohols 15 bis 60 Gew.-% beträgt.

10. Silikon-in-Wasser-Kosmetikum nach Anspruch 9, wobei die Menge an Wasser 20 bis 70 Gew.-% beträgt.

11. Silikon-in-Wasser-Kosmetikum nach Anspruch 1, das eine Viskosität von 250 bis 100.000 mPa.s bei 25 °C aufweist.

12. Silikon-in-Wasser-Kosmetikum nach Anspruch 1, wobei der Gehalt des hydrophilen Polymers 0,001 bis 5,0 Masse-% beträgt.

13. Silikon-in-Wasser-Kosmetikum nach Anspruch 1, wobei die Mischung aus Wasser, polyhydrischem Alkohol, Polyorganosiloxan-Teilchen und hydrophilem Polymer eine Menge von mindestens 90 Gew.-% des Gewichts des Kosmetikums ausmacht.

14. Verfahren zum Herstellen eines transparenten oder durchscheinenden Silikon-in-Wasser-Kosmetikums nach Anspruch 1, wobei das Verfahren einen Schritt des Herstellens (a) eines wässrigen Mediums durch Mischen von Wasser und einem ersten polyhydrischen Alkohol, einen Schritt des Zugebens (b) einer Polyorganosiloxan-Dispersion aus in Wasser dispergierten Polyorganosiloxan-Teilchen und eines zweiten polyhydrischen Alkohols, der identisch oder anders als der erste polyhydrische Alkohol sein kann, zu dem wässrigen Medium umfasst, wobei ein Unterschied im Brechungsindex zwischen dem (a) wässrigen Medium und der (b) Polyorganosiloxan-Dispersion innerhalb von ± 0,05 oder vorzugsweise innerhalb von ± 0,01 liegt.

## Revendications

1. Cosmétique de type silicone dans l'eau transparent ou translucide comprenant de l'eau, au moins un alcool polyhydrique, une dispersion de polyorganosiloxane comprenant des particules de polyorganosiloxane encapsulé, et un polymère hydrophile, lesdites particules de polyorganosiloxane comprenant un cœur de polyorganosiloxane recouvert d'une écorce de polymère,
dans lequel le polymère hydrophile est le pullulane.

2. Cosmétique de type silicone dans l'eau selon la revendication 1, lequel cosmétique comprend un milieu aqueux comprenant de l'eau et l'alcool polyvalent, et les particules de polyorganosiloxane sont dispersées dans ce milieu aqueux qui forme une phase continue.

3. Cosmétique de type silicone dans l'eau selon la revendication 2, dans lequel la teneur en le milieu aqueux est de 50 % à 95 % en masse par rapport à la masse totale du cosmétique.

4. Cosmétique de type silicone dans l'eau selon la revendication 1, dans lequel l'alcool polyvalent est un glycérol.

5. Cosmétique de type silicone dans l'eau selon la revendication 1, dans lequel le rapport en masse de l'eau à l'alcool polyvalent est compris entre 0,30 et 8,0.

6. Cosmétique de type silicone dans l'eau selon la revendication 2, dans lequel le rapport en masse de la dispersion de polyorganosiloxane au milieu aqueux est de 0,05 à 1,0 partie en masse.

7. Cosmétique de type silicone dans l'eau selon la revendication 1, dans lequel le cœur de polyorganosiloxane est un polymère réticulé de diméthicone/vinyldiméthicone, et l'écorce de polymère comprend un polymère cationique et un polymère anionique.

8. Cosmétique de type silicone dans l'eau selon la revendication 1, dans lequel les particules de polyorganosiloxane représentent de 10 % à 20 % en poids.

9. Cosmétique de type silicone dans l'eau selon la revendication 1, dans lequel la quantité d'alcool polyvalent est de 15 % à 60 % en poids.

10. Cosmétique de type silicone dans l'eau selon la revendication 9, dans lequel la quantité d'eau est de 20 % à 70 % en poids.

11. Cosmétique de type silicone dans l'eau selon la revendication 1, ayant une viscosité de 250 à 100 000 mPa.s à 25°C.

12. Cosmétique de type silicone dans l'eau selon la revendication 1, dans lequel la teneur en polymère hydrophile est de 0,001 % à 5,0 % en masse.

13. Cosmétique de type silicone dans l'eau selon la revendication 1, dans lequel le mélange d'eau, d'alcool polyvalent, de particules de polyorganosiloxane et de polymère hydrophile représente une quantité qui est d'au moins 90 % en poids du poids du cosmétique.

14. Procédé pour préparer un cosmétique de type silicone dans l'eau transparent ou translucide selon la revendication 1, ledit procédé comprenant une étape de préparation (a) d'un milieu aqueux par mélange d'eau et d'un premier alcool polyvalent, une étape d'addition (b) d'une dispersion de polyorganosiloxane de particules de polyorganosiloxane dispersées dans de l'eau et un deuxième alcool polyvalent qui peut être identique au ou différent du premier alcool polyvalent, au milieu aqueux, dans lequel la différence d'indice de réfraction entre (a) le milieu aqueux et (b) la dispersion de polyorganosiloxane est de l'ordre de ± 0,05, ou de préférence de l'ordre de ± 0,01.
